# EUROPEAN PATENT APPLICATION

(11) **EP 2 140 911 A1**
(43) Date of publication of application: **06.01.2010**
(21) Application number: 09251727.5
(22) Date of filing: 03.07.2009
(51) Int. Cl.: A61N 5/06, A61H 11/00

(54) **Hairbrush providing multiple hair growth stimulating therapies**

(30) Priority: 03.07.2008 US 78208 P
(71) Applicant: Actervis Gmbh, 6341 Baar (CH)
(72) Inventor: Torres Martin, Juan Ramon, Cerdanyola Del Valles, Barcelona (ES)
(74) Representative: Wightman, David Alexander

(57) **Abstract**

Hair growth therapy is provided by a device capable of delivering multiple hair growth therapies to a user, either individually or in combination. The device includes multiple optical sources 22,26,28,30 providing an array of different optical stimulation signals, while also having vibration capabilities to provide physical stimulation. The multiple optical stimulation therapies include high powered lasers 22, and light emitting diodes (LEDs) 26,28,30 of different wavelengths. Through the use of these multiple light sources, stimulation of hair follicles is provided at the root, along the upper portion, and throughout the base, each promoting healthy hair growth. In addition, the vibration therapies provide a massaging affect, thus further stimulating blood flow in the scalp and helping to support healthy hair growth. The device is configured to resemble a hairbrush 10, having brushing extensions or bristles 40 at one end, thus allowing the user to simultaneously brush through existing hair while also applying the therapies outlined above. Lastly, the bristles 40 are removable, thus allowing these same therapies to be applied to those bald individuals or individuals having extremely thin hair.

## Description

The present invention relates to devices and therapies which promote the growth of hair. More specifically, the present invention provides a device which combines multiple therapies which promote hair growth, including multiple types of optical stimulation along with massage therapy.

Hair loss is a troubling issue for many individuals. In response, several therapies and approaches exist to promote the growth of hair for individuals who are dealing with hair loss either naturally or due to various medical conditions. These approaches include the use of lotions, or crèmes, surgeries, etc. While several of these approaches provide various levels of success, there exists a continuing need for products which stimulate natural hair growth.

The present invention helps to promote growth of human hair by applying multiple therapies including massage or physical stimulation and/or optical stimulation. Further, the optical stimulation utilized preferably includes a combination of multiple light signals, each designed to produce a specific desired result.

In one embodiment of the present invention, a hairbrush is designed to have a plurality, typically three, different types of light sources contained therein. In addition a vibration feature may be designed to massage the user's scalp. By providing an embodiment that preferably also includes removable bristles, the brush is also appropriate for persons with very little hair, as it can provide therapy without scratching or disturbing existing hair follicles.

The multiple optical signals utilized by the brush preferably include an LED signal, preferably employing different types of LEDs having different wavelengths. A first type of LED may be specifically designed to produce a relatively deep penetrating optical signal. In the preferred embodiment, this deep penetrating optical signal is produced by one or more LEDs, preferably a number of red LEDs, with each producing energy having a wavelength of 640 to 680 nm, preferably an approximate wavelength of 660 nm. Similarly, a second type of LED may be utilized to produce a surface stimulating optical signal. In the preferred embodiment, this second optical signal is produced by one or more LEDs, preferably a number of blue LEDs, each producing energy at a wavelength of 450 to 490 nm, preferably a wavelength of approximately 470 nm. Both the red LEDs and blue LEDs are preferably arranged within the housing of a brush, and may be positioned in the same general area as a number of combing bristles which extend outwardly. Thus optical therapy can be easily applied while simultaneously brushing existing hair.

In addition to the various types of energy produced by the above mentioned LEDs, the present invention preferably also includes at least one high energy laser for producing a third optical signal. This laser is preferably positioned behind a window within the brush housing which generally applies laser energy to the root of the hair, thus further promoting hair growth. In the preferred embodiment, the laser preferably has a wavelength of 600 to 700 nm, preferably a wavelength of approximately 650 nm.

Lastly, the brush portion is preferably designed to have a selectable vibration feature to provide physical stimulation to the scalp when used. Vibration, and the related massaging of the scalp is intended to increase blood flow and thus further promote the growth of hair in the related areas.

To provide ease of use, the present invention is preferably configured in a generally well accepted brush format, having a handle end and a bristled end. To provide additional flexibility, the bristled end is preferably configured to allow the bristles or brushing extensions to be easily removed. Removal of the bristles allows the various types of stimulation outlined above to also be used on bald or very thin haired individuals without damage to their scalp or existing hair follicles.

The bristles or brushing extensions may be provided by a removable brushing attachment configured to be attached to an operative end portion of the brush such that the bristles or brushing extensions extend generally transverse to the operative end portion. The bristles or brushing extensions may be substantially rigid elements having sufficient rigidity to transfer vibration therapy from the main body to the users scalp. Alternatively, the bristles or brushing extensions may be flexible elements having sufficient flexibility to bend slightly in response to hair brushing pressure while also being capable of transferring vibration therapy from the main body to the users scalp. The bristles or brushing extensions may have no alignment or relation to the light sources.

The operative end portion may have a substantially smooth light emitting surface when the brushing attachment is removed, with the laser, the or each first LED and the or each second LED all being recessed behind the light emitting surface, thereby allowing the smooth surface to be placed in direct contact with the users scalp.

In one embodiment the present invention provides a hair growth brush for providing multiple hair growth therapies to the scalp of a user, the brush comprising a main body having a handle portion and an operative end portion, the main body being elongated with a central axis, a removable brushing attachment having a plurality of brushing elements extending from a surface thereof and a plurality of openings, the brushing attachment configured to be attached to the operative end portion of the main body such that the brushing elements extend in a direction generally transverse to the central axis, a laser, a first LED and a second LED mounted within the operative end of the main body to provide optical hair growth therapy to the user, each of the laser, the first LED and the second LED being aligned with a respective one of the plurality of openings in the removable brushing attachment when attached, the first LED and the second LED having different operative wavelengths thus providing optical signals which are different from one another, a vibration generator located within the main body to generally provide vibration to the operative end and thereby provide massage therapy to the scalp of the user, and control means located on the handle portion to selectively energize the laser, the first LED, the second LED and the vibration generator.

The control means may comprise a plurality of switches including a first switch to simultaneously power the laser, the first LED and the second LED, and a second switch to power the vibration generator. A power indicator light may be positioned on the handle portion to verify that the laser, first LED and second LED is operating.

In another embodiment the present invention provides a brush for providing hair growth therapy to a user's scalp, the brush comprising a housing having a handle for a user to grab, a plurality of removably attached brushing extensions positioned away from the handle, where the extensions are configured along an active surface of the housing so as to act as a hair brushing structure, a plurality of red LEDs positioned on the active surface of the housing so as to provide optical signals in the direction away from the active surface and in the direction of the user's scalp when in use, a plurality of blue LEDs positioned on the active surface of the housing so as to provide optical signals in the direction away from the active surface and in the direction of the user's scalp when in use, and at least one laser positioned within the housing and aligned behind a window which exists in the active surface, with the window being centrally positioned and surrounded by the brushing extensions and the LEDs, the laser and accompanying window capable of applying a produced laser signal to the user's scalp.

Preferably, the brush further comprises a vibration motor positioned within the housing to provide vibration of the housing and allow vibration therapy to be transmitted to the user's scalp through the brushing extensions.

In another embodiment the present invention provides a hair growth brush for providing optical and massage hair growth therapies to the scalp of a user, the brush comprising, an elongated main body having a handle portion and an operative end portion, the handle portion including a gripping structure and a plurality of controls, the elongated main body having a central axis extending through a center thereof, a removable brushing attachment having a plurality of brushing elements extending from a surface thereof, a central window and a plurality of openings adjacent the central window, the brushing attachment configured to be attached to the operative end portion of the main body such that the brushing elements extend in a direction generally transverse to the central axis, a laser, a plurality of first LEDs and a plurality of second LEDs mounted within the operative end of the main body to provide the optical hair growth therapy to the user, the laser being centrally located and aligned with the window of the brushing attachment when attached and the plurality of first LEDs and the plurality of second LEDs being aligned adjacent the laser substantially parallel with the central axis, the plurality of first LEDs and the plurality of second LEDs further being aligned with respective openings in the removable brushing attachment when attached, the plurality of first LEDs and the plurality of second LEDs having different operative wavelengths thus providing optical signals which are different from one another, and a vibration generator located within the main body to generally provide vibration to the operative end and thereby provide massage therapy to the scalp of the user, wherein the plurality of controls located on the handle portion include an optical power switch to selectively energize the laser, the first plurality of LEDs, the second plurality of LEDs and a vibration activation switch to energize the vibration generator, and wherein the handle portion further has an indicator light to indicate that the laser, first plurality of LEDs, the second plurality of LEDs are powered.

By combining the various therapies addressed above, the device of the present invention provides a unique and effective solution for those dealing with hair loss. While each individual therapy alone is known to be beneficial, the combination of all four creates a very effective and valuable device.

Further advantages of the present invention can be seen in the attached drawings and attachments, in which:

Fig. 1 is an exploded view illustrating brush side of the device;

Fig. 2 is a second exploded perspective view illustrating the removable comb portion; and

Fig. 3 is a top perspective view of the device.

The discussion below highlights features of the present invention by describing a preferred embodiment in considerable detail. This discussion is not intended to be limiting in any way, and various features and characteristics may be modified or changed while continuing to be within the scope of the present invention.

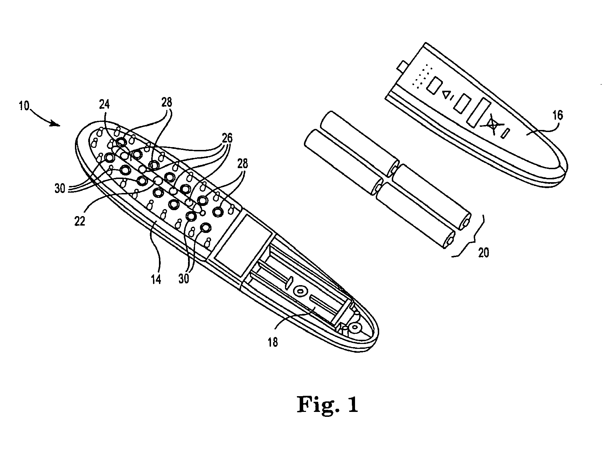

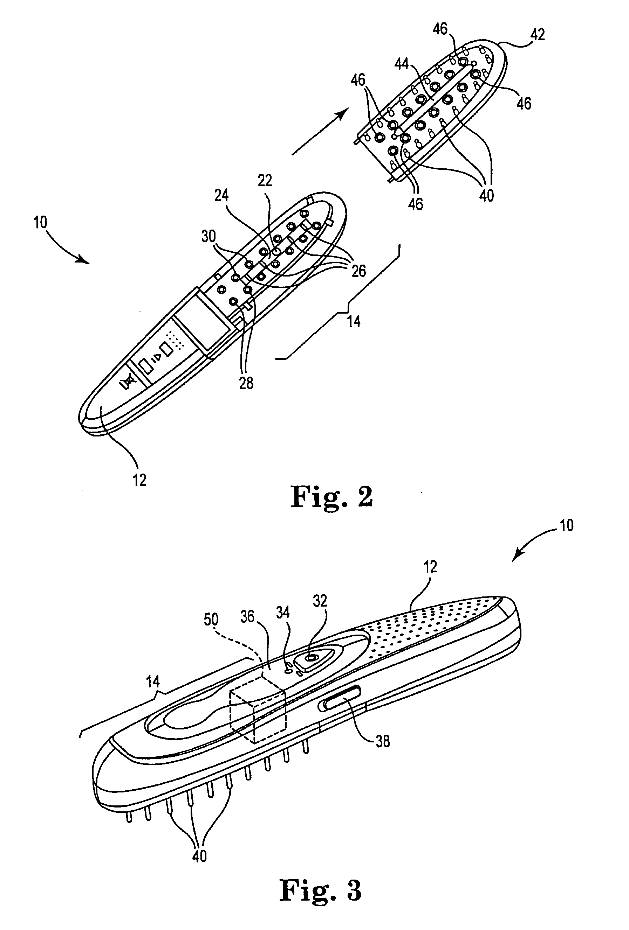

Referring now to Figs. 1 - 3, a multi-therapy brush 10 is illustrated from various perspectives. As better illustrated in Figs. 2 and 3, multi-therapy brush 10 includes a main body having a handle portion 12 and an operative end portion14. As shown the main body is elongated with a central axis. As recognized, handle portion 12 simply includes an extension or handle for the user to hold and appropriately operate, for example, while brushing or combing hair. As shown in Fig. 1, handle portion 12 also includes a removable battery cover 16 which is typically attached above a battery carrying portion 18. A number of batteries 20 are placed in multiple-therapy device 10 to provide appropriate power (in this embodiment, four batteries are used to provide an appropriate level of power).

Referring more specifically to operative end portion 14 shown in the figures, several different components and features are evident. As suggested above, the present invention utilizes multiple light sources in conjunction with vibration mechanisms to provide multiple therapies using one single device. As illustrated in Fig. 1, operative end portion 14 houses a number of different light sources on an active surface thereof. More specifically, at least one laser 22 is centrally located within operative end portion 14 along with a number of LEDs. In one embodiment, laser 22 is positioned behind a window 24 which occupies a central region of operative end portion 14, preferably substantially parallel to the central axis. Also positioned behind window 24 are a plurality, in this case four, internal red LEDs 26, with each LED capable of providing an optical signal through window 24. It is contemplated that alternative embodiments would include one or more additional lasers in place of the four internal red LEDs 26, with the additional lasers thus producing additional laser energy to slightly alter the therapy provided. This could include two or more lasers, depending on the amount and orientation of laser energy desired. Also included within operative end 14 are a plurality of blue LEDs 28 extending down a first side adjacent to window 24, preferably aligned in a direction substantially parallel to the central axis, and a plurality of red LEDs 30, also positioned adjacent to window 24, preferably aligned in a direction substantially parallel to the central axis, and preferably opposite blue LEDs 28. Utilizing the aforementioned combination of LEDs and lasers, multiple light sources are produced by the multi-therapy device 10 of the present invention. It is contemplated that the number and type of LEDs and/or lasers employed and the arrangement thereof may be varied from that shown.

In one embodiment (as suggested above), these multiple light sources are intended to be blue LEDs producing an optical signal having a wavelength of 450 to 490 nm, preferably of approximately 470 nm, red LEDs producing an optical signal having a wavelength of 640 to 680 nm, preferably of approximately 660 nm, and at least one laser producing an optical signal having a wavelength of 600 to 700 nm, preferably of approximately 650 nm. In this embodiment, the laser is a 4.5 mW Class 3R certified laser having a specified wavelength of 650 mn +/- 5 nm. Similarly, the blue LEDs have a specified wavelength of 470 nm +/- 5 nm and the red LEDs have a specified wavelength of 660 nm +/- 5 nm. Each of these optical signals will provide different types of stimulation when applied to a user's scalp. Specifically, the optical signals of approximately 660 nm wavelength (red LED signals) stimulate deep into the scalp, intending to provide stimulation to the base of hair follicles. In a similar manner, the blue LED signals (optical signals of approximately 470 nm wavelength) are intended to stimulate the user's hair closer to the scalp. Additionally, the high precision laser beam is directed towards the root of the hair and is intended to stimulate the cells of the hair follicles and make hair thicker. This combination of three separate light signals will increase hair strength and stop hair loss by providing multiple types of optical stimulation, with each type strengthening the hair in different manner.

The multi-therapy brush 10 further includes one or more controls to selectively energize the laser, LEDs and a vibration generator (discussed below). In this embodiment the controls include a power switch 32 and a power indicator light 34 positioned on a back side 36. Additionally, a vibration control switch 38 is shown on a side of the device (used to conveniently power the vibration features discussed below). Each of these control switches are conveniently positioned to allow the user to operate the multi-therapy device. It will be understood that any suitable control means may be employed, for example a rotatable control knob, for selecting different functions individually or in combination.

Also contained within multi-therapy brush 10 is a vibration-producing mechanism 50 shown generally in Fig. 3. Generally speaking, this mechanism provides vibration to the entire device, which then can be transferred to the user's scalp. When the vibration mechanism is operating, brushing extensions 40 will also necessarily vibrate, thus providing a massaging therapy to the scalp. This massage therapy will stimulate blood flow throughout the scalp, and specifically around the hair follicles, thus further promoting hair growth. The vibration mechanism 50 can be an electric motor, or similar piezo electric element capable of producing the desired vibration.

As shown in the various Figures, multi-therapy device 10 includes a number of brushing extensions or pins 40 which can be utilized in a typical combing or brushing manner. The brushing extensions or pins 40 are positioned on the operative end with no alignment or relation to the light sources. The brushing extensions or pins 40 extend away from the main body, preferably transverse to the central axis, so that vibration therapy can be transferred from the main body to the user's scalp. Referring specifically now to Fig. 2, the brushing extensions 40 or brush end of the present multi-therapy device 10 is specifically designed to be removable. Specifically, a removable attachment member 42 provides support for brushing extensions 40. As also shown, attachment member 42 also includes a central window portion 44 and a plurality of openings 46 adjacent central window 44.

When attached, the removable attachment member 42 is configured to allow optical signals produced by the various light sources to pass through openings 46 or central window 44 and be applied to a user's scalp. When the removable attachment member 42 is removed, the sources of optical energy (LEDs and at least one laser) are exposed and unobstructed. Thus, the device can be utilized without attachment member 42. The removal of attachment member 42 would be helpful for balding individuals, or those with very thin hair. In this particular use the multi-therapy device could simply expose the hair or scalp to the optical signals, thus providing therapy without the brushing capabilities. When detached, the operative end portion preferably has a substantially smooth light emitting surface with the light sources preferably recessed behind the light emitting surface allowing the smooth surface to be placed in direct contact with the users scalp.

Those skilled in the art will further appreciate that the present invention may be embodied in other specific forms without departing from the spirit or central attributes thereof. In that the foregoing description of the present invention discloses only exemplary embodiments thereof, it is to be understood that other variations are contemplated as being within the scope of the present invention. Accordingly, the present invention is not limited in the particular embodiments which have been described in detail herein and extends to include any of features described herein separately or in combination with any other feature. Rather, reference should be made to the appended claims as indicative of the scope and content of the present invention.

## Claims

1. A hair growth brush for providing multiple hair growth therapies to the scalp of a user, the brush comprising:
a main body having a handle portion (12) and an operative end portion (14), the main body being elongated with a central axis;
a removable brushing attachment (42) having a plurality of brushing elements (40) extending from a surface thereof and a plurality of openings (44,46), the brushing attachment (42) configured to be attached to the operative end portion (14) of the main body such that the brushing elements (40) extend in a direction generally transverse to the central axis;
a laser (22), a first LED (28) and a second LED (30) mounted within the operative end (14) of the main body to provide optical hair growth therapy to the user, each of the laser (22), the first LED (28) and the second LED (30) being aligned with a respective one of the plurality of openings (44,46) in the removable brushing attachment (42) when attached, the first LED (28) and the second LED (30) having different operative wavelengths thus providing optical signals which are different from one another;
a vibration generator (50) located within the main body to generally provide vibration to the operative end (14) and thereby provide massage therapy to the scalp of the user; and
control means (32,38) located on the handle portion (12) to selectively energize the laser (22), the first LED (28), the second LED (30) and the vibration generator (50).

2. The hair growth brush of claim 1 wherein the first LED (28) generates a blue light signal having a wavelength of 450 to 490 nm and the second LED (30) generates a red light signal having a wavelength of 640 to 680 nm.

3. The hair growth brush of claim 1 or claim 2 wherein the laser (22) is centrally located in the operative end (14) and provides a light signal having a wavelength of 600 to 700 nm.

4. The hair growth brush of claim 1 or claim 3 further comprising a plurality of additional first LEDs (28) aligned in a direction substantially parallel to the central axis and positioned adjacent the laser (22), and a plurality of additional second LEDs (30) aligned in a direction substantially parallel to the central axis and positioned adjacent the laser (22) and opposite the plurality of first LEDs (28).

5. The hair growth brush of claim 4 wherein the operative end (14) has a substantially smooth light emitting surface, with the laser (22), the plurality of first LEDs (28) and the plurality of second LEDs (30) all being recessed behind the light emitting surface, thereby allowing the smooth surface to be placed in direct contact with the users scalp when the brushing attachment (42) is removed.

6. The hair growth brush of any preceding claim wherein the brushing elements (40) are substantially rigid elements extending away from the main body having sufficient rigidity to transfer vibration therapy from the main body to the users scalp.

7. The hair growth brush of any of claims claim 1 to 5 wherein the brushing elements (40) are flexible elements extending away from the main body having sufficient flexibility to bend slightly in response to hair brushing pressure while also being capable of transferring vibration therapy from the main body to the users scalp.

8. The hair growth brush of any preceding claim wherein the control means comprises a plurality of switches (32,38) including a first switch (32) to simultaneously power the laser (22), the first LED (28) and the second LED (30), and a second switch (38) to power the vibration generator (50).

9. The hair growth brush of claim 8 further having a power indicator light (34) positioned on the handle portion (12) to verify that the laser (22), first LED (28) and second LED (30) is operating.

10. The hair growth brush of any preceding claim wherein one of the plurality of openings in the removable brushing attachment (42) is filled with a window element (44), and wherein the laser (22) is positioned behind the window element (44).

11. The hair growth brush of claim 1 or claim 4 wherein the operative end (14) further has a plurality of additional light sources (26) centrally located and adjacent the laser (22), such that the laser (22) and the additional light sources (26) are aligned substantially parallel with the central axis and wherein one of the plurality of openings in the removable brushing attachment (42) is filled with a window element (44) which is positioned and aligned such that the laser (22) and additional light sources (26) are positioned behind the window element (44).

12. The hair growth brush of claim 11 wherein the additional light sources are red LEDs (26) having an operative wavelength of 640 to 680 nm.

13. The hair growth brush of claim 4 wherein the plurality of first LEDs (28) each generate a blue light signal having a wavelength of 450 to 490 nm, the plurality of second LEDs (30) each generate a red light signal having a wavelength of 640 to 680 nm, and the laser (22) provides a light signal having a wavelength of 600 to 700 nm.

14. The hair growth brush of claim 1 or claim 4 wherein the operative end (14) further has a plurality of additional light sources (26) centrally located and adjacent the laser (22), such that the laser (22) and the additional light sources (26) are aligned substantially parallel with the central axis and behind a window element (44) when the brush attachment (42) is attached, the additional light sources (26) being red LEDs having an operative wavelength of 640 to 680 nm.

15. The hair growth brush of claim 1 or claim 4 wherein the operative end (14) has a substantially smooth light emitting surface when the brushing attachment (42) is removed, with the laser (22), the or each first LED (28) and the or each second LED (30) all being recessed behind the light emitting surface, thereby allowing the smooth surface to be placed in direct contact with the users scalp.
